# EUROPÄISCHE PATENTANMELDUNG

(11) **EP 0 965 647 A1**
(43) Veröffentlichungstag der Anmeldung: **22.12.1999**
(21) Anmeldenummer: 98110608.1
(22) Anmeldetag: 10.06.1998
(51) Int. Cl.: C12Q 1/68

(54) **Vorrichtung zur parallelen Identifizierung und Quantifizierung von Polynukleinsäuren**

(71) Anmelder: Memorec Medical Molecular Research Cologne Stoffel GmbH, 50829 Köln (DE)
(72) Erfinder: Bosio, Andreas, Dr., D-50674 Köln (DE); Stoffel, Markus, Prof.Dr., New York, NY 10021 (US); Stoffel, Wilhelm, Prof.Dr.Dr., D-50933 Köln (DE)
(74) Vertreter: Meyers, Hans-Wilhelm, Dr.Dipl.-Chem.

(57) **Zusammenfassung**

Träger mit an mindestens einer Hauptoberfläche des im wesentlichen planaren Trägers kovalent gebundener Oligo- oder Polynukleinsäure, dadurch gekennzeichnet, daß
die Hauptoberfläche des Trägers eine reaktive Gruppe aufweist, die mit einem bifunktionellen Spacer unter Ausbildung einer kovalenten Bindung zwischen einer funktionellen Gruppe des Spacers und der reaktiven Gruppe der Hauptoberfläche des Trägers reagiert hat,
die zweite funktionelle Gruppe des bifunktionellen Spacers mit einer der funktionellen Gruppen eines bifunktionellen Linkers reagiert und
die zweite funktionelle Gruppe des bifunktionellen Linkers mit dem Oligo- oder Polynukleotid, das kovalent gebunden werden soll, unter Ausbildung einer kovalenten Bindung am 5'- oder 3'-Terminus des Oligo- oder Polynukleotids reagiert hat.

## Beschreibung

Die Erfindung betrifft eine Vorrichtung gemäß Anspruch 1, Verwendung des erfindungsgemäßen Trägers sowie Herstellung des erfindungsgemäßen Trägers.

Analysen, die auf molekularbiologischer Ebene durchgeführt werden, gewinnen zunehmend an Bedeutung. In den meisten Fällen wird in solchen Methoden ein zu analysierendes Nukleinsäuregemisch durch Hybridisierungsreaktionen mit sogenannten Sonden hybridisiert und charakterisiert. Insbesondere bei Fragestellungen, bei denen gleichzeitig eine Vielzahl von Polynukleinsäuren verschiedener Art nachgewiesen werden sollen, kommt es zu methodischen Engpässen. Man versucht insbesondere durch parallele Prozeßführung eine Vielzahl von Analyseschritten in kurzer Zeit durchzuführen. Dabei stellt sich oftmals das Problem, daß Trägersysteme, auf denen die Hybridisierungsexperimente durchgeführt werden können, nur beschränkte Raumkapazität aufweisen. Man versucht daher durch Verwendung von Trägern, auf denen eine Vielzahl von Proben plaziert werden können, diese Probleme zu lösen. Insbesondere werden im Stand der Technik Träger beschrieben, die Mikro- oder Nanokompartimente aufweisen zur Aufnahme entsprechend kleiner Volumina, der meistens in Lösung befindlichen Analyten. Entsprechende Trägersysteme sind beispielsweise durch Ätzen von Oberflächen von aus Silizium aufgebauten Wafern erhältlich.

Solche Systeme sind üblicherweise nur recht aufwendig herstellbar und erreichen oft eine nicht zufriedenstellende Kapazität von Probenkompartimenten, die für eine entsprechende Parallelisierung notwendig wären.

Aufgabe der vorliegenden Erfindung ist mithin die Bereitstellung eines Trägers, der die genannten Nachteile des Standes der Technik vermeidet. Insbesondere soll der erfindungsgemäße Träger Nukleinsäuren, bevorzugt mit definierter Sequenz und möglichst gleicher Länge von 200 bp bis 400 bp in hoher Dichte binden können und eine hohe Parallelisierung von zu untersuchenden Proben erlauben.

Erfindungsgemäß gelöst wird diese Aufgabe durch einen Träger mit den Merkmalen des Patentanspruchs 1. Bevorzugte Weiterbildungen des erfindungsgemäßen Trägers finden sich in den Ansprüchen 2 bis 8. Gegenstand der vorliegenden Erfindung ist ebenfalls ein Verfahren zur Herstellung des erfindungsgemäßen Trägers sowie dessen Verwendung.

Der erfindungsgemäße Träger mit an mindestens einer Hauptoberfläche des im wesentlichen planaren Trägers kovalent gebundener Oligo- oder Polynukleinsäuren ist dadurch gekennzeichnet, daß die Hauptoberfläche des Trägers eine reaktive Gruppe aufweist, die mit einem bifunktionellen Spacer unter Ausbildung einer kovalenten Bindung zwischen einer funktionellen Gruppe des Spacers und der reaktiven Gruppe der Hauptoberfläche des Trägers reagiert hat. Unter Hauptoberfläche wird jede Oberfläche verstanden, die eine hinreichende Dimension aufweist, um eine für die Verwendung des Trägers notwendige Anzahl von Proben aufzunehmen.

Die zweite funktionelle Gruppe des bifunktionellen Spacers hat mit einer funktionellen Gruppen eines bifunktionellen Linkers reagiert und die zweite funktionelle Gruppe des bifunktionellen Linkers hat mit dem Oligo- oder Polynukleotid, das kovalent gebunden werden soll (Leit-Nukleinsäure), unter Ausbildung einer kovalenten Bindung am 5'- oder 3'-Terminus des Oligo- oder Polynukleotids reagiert.

Vorzugsweise ist das Polynukleotid eine RNA, DNA oder PNA. Der Träger besteht vorzugsweise aus einem Glas oder einem anderen hauptsächlich aus Siliziumoxid bestehenden Material. Vorzugsweise weist der bifunktionelle Spacer, der an die Hauptoberfläche des erfindungsgemäßen Trägers gebunden ist, die nachstehende Struktur auf
(XO)₃-Si-Y-Nu
wobei
X = C₁-C₃ Alkyl,
Y = C₂-C₄ Alkylen,
Nu = eine nukleophile Gruppe wie -NH₂, -NHR, mit
R = -CH₂-CH₂-NH₂, -CH₂-CH₂-NH -CH₂-CH₂-NH_{2,}-CO-NH₂, oder SH, ist.

Insbesondere bevorzugt ist ein Spacer der Struktur
Me₃OSi-CH₂-CH₂-CH₂-NH₂.

Der Linker, der mit der noch freien Gruppe des bifunktionellen Spacers eine kovalente Bindung eingeht, hat die folgende Struktur
E₁-A-E₂,
wobei
E₁ und E₂ gleiche oder verschiedene elektrophile Gruppen wie
S = C = N- sind und
A = C₂-C₃₀ Alkyl, Heteroalkyl, Aryl, Heteroaryl, ist.

Insbesondere bevorzugt ist ein Linker mit der folgenden Struktur
S = C = N-Phenylen-N = C = S.

Der erfindungsgemäße Träger weist vorzugsweise ein kovalent an den bifunktionellen Linker gebundenes Oligo- oder Polynukleotid auf, wobei die kovalente Bindung mittels einer am 3'- oder 5'-Terminus über ein Alkan mit einer Länge von 4 bis 30 Methylengruppen oder über einen Polyether mit 2 bis 20 wiederholenden Struktureinheiten synthetisch oder über die PCR Reaktion angefügte primäre Aminogruppe gebunden ist.

E.M. Southern (E.M. Southern et al. (1992), Nucleic Acids Research **20**: 1679 bis 1684 und E.M. Southern et al. (1997), Nucleic Acids Research **25**: 1155 bis 1161) beschreibt die Herstellung sogenannter Oligonukleotid-Anordnungen durch direkte Synthese an einer Glasoberfläche, die mit 3-Glycidoxypropyltrimethoxysilan und anschließend mit einem Glycol derivatisiert wurde.

Ein ähnliches Verfahren betrifft die Veröffentlichung von S.P.A. Fodor (Pease, A.C. et al. (1994), Proc. Natl. Acad. Sci. USA **91**: 5022 bis 5026). Die dort beschriebene *in situ* Oligonukleotidsynthese findet mittels vollautomatischer, lichtgesteuerter kombinatorischer Chemie statt. Die Direktsynthese von Oligonukleotiden auf einer Glasoberfläche erlaubt eine maximale Länge von ca. 30 Basen. Eine Sicherstellung des Syntheseverlaufs der individuellen Sequenz längerer Oligonukleotide ist - wenn überhaupt - mit einem nicht vertretbaren Aufwand verbunden. Diese Oligonukleotide als Leit-DNA erlauben die Hybridisierung eines nur sehr kurzen Stücks der Analyse-Nukleinsäure. Um diesen Nachteil zu umgehen, werden für jede zu analysierende Nukleinsäure mehrere Oligonukleotide als Leit-DNAs synthetisiert. Dies hat eine größere Platzbeanspruchung und damit größeres Probenvolumen an Analyse-Nukleinsäure zur Folge. Die geringe Länge der Oligonukleotide schließt ferner Kreuzhybridisierungen mit verschiedenen Analyse-Nukleinsäuren nicht aus. Dadurch wird eine eindeutige Zuordnung der aufgenommenen Signale erschwert.

P.O. Brown (DeRisi et al. (1997), Science **278**: 680-686) offenbart zur Herstellung sogenannter DNA-Chips Polylysin beschichtete Glasoberflächen, auf die kleinste DNA-Mengen mittels Kapillartechnik aufgetropft werden. Die Immobilisierung der Leit-DNA auf einer Polylysin-Oberfläche beeinträchtigt allerdings die Hybridisierung und setzt damit die Nachweisgrenze und Verläßlichkeit bei der Detektion der Analyse-Nukleinsäuren beträchtlich herab.

L.M. Smith (Guo, Z. et al. (1994), Nucleic Acids Research **22**: 5456-5465) beschreibt eine Technik zur Immobilisierung von Oligonukleotiden, die darauf beruht, daß Oligonukleotide mit einer 5' terminalen Aminogruppen derivatisiert werden und diese dann auf eine Glasoberfläche gebracht werden, die mit 3-Aminopropyltrimethoxysilan und anschließend mit 1,4-Phenyldiisothiocyanat derivatisiert wurde. Während die Chemie zur Immobilisierung der Oligonukleotide die Nachteile der zuvor beschriebenen Systeme umgeht, gelten auch hier die zuvor erwähnten Nachteile, die durch die Verwendung von kurzen Oligonukleotiden als Leit-DNA bedingt sind.

200 bis 400 bp lange DNA Fragmente bilden aus folgenden Gründen bevorzugte Leit-DNAs: Die Länge und die damit gegebene Schmelztemperatur sind ausreichend, um - bei sorgfältiger Auswahl und einer maximalen Komplexität wie sie das menschliche Genom darstellt - mit hoher Sicherheit eine nicht redundante Hybridisierung zu garantieren.

Die Kürzesten bekannten cDNAs liegen im Bereich von ca. 200 bp. Somit können alle cDNAs einer zu analysierenden cDNA-Population vollständig oder als 200 bis 400 bp-Fragmente an die Festphase gebunden werden. Die ähnliche Länge aller aufgetragenen DNAs ergibt bei der Hybridisierung mit einer markierten Analyse-Nukleinsäure Hybridisierungssignale, die nicht durch die Länge oder unterschiedliche Hybridisierungskinetik der Leit-Nukleinsäure beeinträchtigt werden.

Die Sequenzen der aufzutragenden Leit-DNAs werden vorzugsweise jeweils einzeln, z.B. durch eine eigens hierfür hergestellt Software, insbesondere aus den öffentlich zur Verfügung stehenden Gen-Datenbank herausgesucht. Es ist bevorzugt die Leit-DNAs auf Nicht-Redundanz zu prüfen. Damit ist weitestgehend ausgeschlossen, daß eine Leit-DNA mit mehreren Analyse-Nukleinsäuren hybridisiert und so zu falsch positiven Signalen führen kann. Die Leit-DNA wird, z.B. mittels RT-PCR und sequenzspezifischer Primer, ausgehend von Gesamt-RNA nach gängigen Protokollen, amplifiziert.

Bei der Auswahl der sequenzspezifischen Primer werden diese vorzugsweise so gewählt, daß sie zur Amplifizierung der gewünschten Leit-Nukleinsäure verschiedener Spezies wie z.B. human und murin geeignet sind. Auch bei diesem Vorgang hat sich eine Länge der Leit-Nukleinsäure von 200 bis 400 bp als geeignet erwiesen. Die Länge reicht aus um in ca. 70 bis 80% der Fälle 18 bis 22 bp lange Primer zu definieren, die mit maximal 3 "mismatch-Basen" die Amplifizierung der Leit-Nukleinsäure beider Spezies erlaubt.

Als Träger werden vorzugsweise an sich bekannte Glas-Objektträger benutzt. Im Gegensatz zu oftmals verwendeten Nylonmembranen bieten z.B. Objektträger den Vorteil, daß sie aufgrund ihrer Starrheit und der Tatsache, daß Glas für die meisten Reagenzien inert ist, wesentlich leichter zu bearbeiten und anschließend von unspezifischen Hybridisierungssignalen freizuwaschen sind. Ein großer Vorteil liegt weiterhin darin, daß Glas zur fluoreszenzgestützten Analyse genutzt werden kann.

Insbesondere die Nutzung piezoelektrischer Nanodispenser zum Auftragen der Leit-DNA auf die Festphase erlaubt eine sehr genaue Dosierung, was für eine verläßliche Quantifizierung der Analyse-DNA bevorzugt ist, die im direkten Zusammenhang mit einer reproduzierbaren Menge an Leit-DNA steht. Die Möglichkeit, Tropfenvolumina von 0,1 nl aufzutragen, erlaubt die Anordnung von 100.000 unterschiedlichen Leit-DNAs z.B. auf der Fläche eines Objektträgers (76 x 26 mm). Damit wird es möglich, auch sehr geringe Nukleinsäuremengen zu detektieren.

Die erfindungsgemäße Immobilisierung der Leit-DNA über die Reaktion eines Isothiocyanats mit einem primären Amin zu N-substituierten Thioharnstoffen birgt Vorteile. Sowohl die Chemikalien als auch die aminomodifizierten 5'-Oligonukleotidprimer zur Synthese der DNAs sind kostengünstig im Vergleich zu anderen Syntheseverfahren, die auf der Phosphoramidit-Chemie beruhen. N-substituierte Thioharnstoffe stellen eine stabile Verbindung dar. Die nach dem hier beschriebenen Verfahren kovalent gebundene DNAs werden auch durch mehrstündiges Kochen in Wasser nicht von der Festphase abgetrennt. Damit können die DNA-Chips auch einer Mehrfachverwendung zugänglich sein. Dies scheitert bei anderen Chips unter anderem daran, daß die für die Regenerierung erforderlichen Waschbedingungen zum Entfernen von spezifischen und unspezifischen Analyse-DNAs aufgrund der Instabilität der Festphasen-Bindung nicht stringent genug gewählt werden können.

Durch die spezifische Bindung der DNA über ihr 5' oder 3' Ende ist weitestgehend sichergestellt, daß nahezu die gesamte Leit-DNA für die Hybridisierung mit der Analyse-DNA zur Verfügung steht und nicht durch unspezifische Bindung an der Oberfläche beeinträchtigt ist. Die DNA-Doppelhelix wird sich aufgrund ihrer recht starren Struktur und negativen Ladung senkrecht zur Festphase ausrichten und so eine maximale Dichte an aufzutragender Leit-DNA ermöglichen.

Die spezifische und monovalente Bindung der Leit-DNA an die Festphase erlaubt nicht zuletzt eine Kontrolle der Menge an aufgetragener DNA über die zur Verfügung stehenden derivatisierbaren Isothiocyanatgruppen.

Die erfindungsgemäße Verwendung eines erfindungsgemäßen Trägers in einem Verfahren zur Identifizierung und Quantifizierung von Polynukleotiden durch eine Markierung der zu analysierenden Polynukleotide und anschließende Hybridisierung auf dem Träger ist ebenfalls Gegenstand der vorliegenden Erfindung.

Die Detektion der Hybridisierung von Leit- und Analyse-Nukleinsäuren kann nach verschiedenen Methoden erfolgen. Eine geeignete Methode besteht in der Markierung der Analyse-Nukleinsäure mittels fluoreszierender Nukleotide und anschließender Detektion der Hybridisierung mittels Fluoreszensmikroskopie. Zur differentiellen bzw. relativen Quantifizierung von Analyse-Nukleinsäuren wird zu der fluoreszenzmarkierten Analyse-Nukleinsäure eine bekannte Menge an Referenz-Nukleinsäure, die einen zweiten Fluoreszenzmarker trägt, zugegeben und für die Hybridisierung auf die immmobilisierte Leit-DNA aufgetragen. Durch Vergleich der detektierten Signalintensitäten erfolgt eine relative Quantifizierung der Analyse-Nukleinsäure.

Das erfindungsgemäße Verfahren zur Herstellung eines erfindungsgemäßen Trägers weist folgende Schritte auf.

Der bifunktionelle Spacer wird in einem polaren aprotischen Lösungsmittel auf die Hauptoberfläche des Trägers aufgebracht, woraufhin gegebenenfalls überschüssiger nicht abreagierter Spacer entfernt wird. Der bifunktionelle Spacer wird beispielsweise in 95%-igem Aceton/Wasser-Gemisch (Vol-%) auf die Hauptoberfläche des Trägers aufgebracht. Vorzugsweise wird der Träger nach den eventuell durchgeführten Waschschritten, insbesondere durch Erhitzen, getrocknet.

Der bifunktionelle Linker wir in einem im wesentlichen wasserfreien polaren aprotischen Lösungsmittel gelöst und zur Reaktion mit dem auf der Hauptoberfläche gebundenen Spacer gebracht. Der Linker sollte vorzugsweise in geringer Konzentration, beispielsweise im Bereich von 0,5 Gew.-% in dem polaren aprotischen Lösungsmittel vorliegen. In diesem Falle kommt z.B. ein Lösungsmittelsystem mit 10% Pyridin/Dimethylformamid (Vol-%) in Betracht. Die Reaktionszeit hängt von der Reaktionsfreudigkeit des bifunktionellen Spacers oder bifunktionellen Linkers ab und kann durchaus mehrere Stunden bei Raumtemperatur oder erhöhter Temperatur betragen. Der Träger kann in diesem Zustand gekühlt und trocken mehrere Monate aufbewahrt werden.

In einem weiteren Ansatz wird das am 5'- oder 3'-Terminus über eine Alkylengruppe mit einer Aminogruppe modifizierte Oligo- oder Polynukleotid in einem Puffer aufgenommen. Hierzu bietet sich insbesondere ein basischer Puffer, beispielsweise ein Carbonatpuffer an. Die Mischung wird auf dem vorher vorbereiteten Träger inkubiert zur Bindung des Oligo- oder Polynukleotids an eine freie Gruppe des bifunktionellen Linkers. Dies kann insbesondere für einen Zeitraum von mehreren Stunden in einer dampfgesättigten Atmosphäre erfolgen. Danach werden eventuell nicht abreagierte Gruppen des bifunktionellen Linkers entfernt. Hierzu dienen insbesondere Amine wie Ethanolamin oder Hydroxylamin. Es handelt sich dabei um dem Fachmann an sich bekannte typische Blockierungsreaktionen reaktiver Gruppen.

Danach wir das auf dem Träger gebundene Oligo- oder Polynukleotid denaturiert. Zur Denaturierung wird beispielsweise der Träger mit dem dann daran kovalent gebundenen Polynukleotid in bidestilliertem Wassern gekocht. Zur Aufrechterhaltung des denaturierten Zustandes kann der Träger mit reinem Alkohol gespült und anschließend kühl und trocken aufbewahrt werden.

Die Erfindung wird anhand der folgenden weiteren Erläuterungen näher beschrieben.

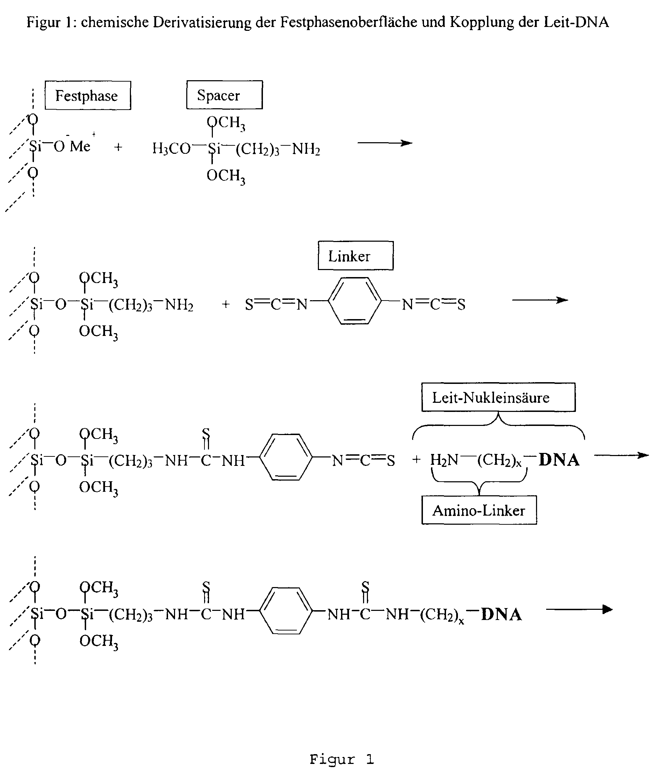

Figur 1: Chemische Derivatisierung der Festphasenoberfläche und kovalente Kopplung der Leit-DNA.

Reinigung der Glas-Objektträger: 76 x 26 mm, reinweißes Glas, ohne Beschichtung, Mattrand oder Beschriftungsfeld; z.B. Fisher Scientific unter der Bezeichnung "Objektträger Reinweiß mit geschnittenen Kanten": zwei Stunden Schütteln der Objektträger in einer Lösung aus 2 N NaOH in 70% EtOH, dreimaliges Waschen mit vollentsalztem (und bidestilliertem) Wasser und einmaliges Waschen mit Aceton.

Beschichtung: Die Objektträger werden zwei Minuten in einer Lösung aus 1% 3-Aminopropyltrimethoxysilan (APTMS) in 95% Aceton/Wasser eingetaucht, dann zehnmal jeweils fünf Minuten in Aceton gewaschen und anschließend 45 Minuten bei 110°C getrocknet.

Derivatisierung der beschichteten Objektträger mit einem Linker: Die Objektträger werden zwei Stunden in einer Lösung aus 0,2 Gew.-% 1,4 Phenyldiisocyanat (PDC) / 10 Vol.-% Pyridin/Dimethylformamid eingetaucht und anschließend mit Methanol und Aceton gewaschen.

Auftragen der Leit-DNA zur kovalenten Kopplung: 0,1 nl PCR-Fragmente werden mittels eines Nano-Dispensers auf definierte Positionen der Objektträger aufgetragen, die Objektträger mindestens eine Stunde bei 37°C in feuchter Atmosphäre inkubiert, dann einmal mit 1% NH₄OH und dreimal mit Wasser gespült und gekühlt und trocken gelagert.

Zur Abtrennung des DNA-Gegenstranges vom Matrizen-Strang werden die Objektträger zehn Minuten bei 96°C in vollentsalztem Wasser inkubiert, mit 96% Ethanol gespült und anschließend bei Raumtemperatur getrocknet. Die Objektträger sind nun für die Hybridisierung mit der Analyse-Nukleinsäure bereit.

Erfindungsgemäß kann auch wie folgt verfahren werden.

Es kann auch eine Glasoberfläche genutzt werden, die nicht zur Nutzung als Objektträger angefertigt wurde.

Die Dichte der derivatisierbaren Aminogruppen auf der Glasoberfläche kann durch die Konzentration der APTMS-Lösung zwischen 0,1 und 10% variiert werden, wodurch eine unterschiedliche Dichte an gekoppelter Leit-DNA eingestellt werden kann.

Die Dichte der derivatisierbaren Aminogruppen kann ferner gesteuert werden durch ein Gemisch aus APTMS/Propyltrimethoxysilan (PTMS) oder APTMS/Tetramethoxysilan (TeMS) im Verhältnis 1:10 bis 10:1.

Vor der Derivatisierung der Aminogruppen mit PDC können die gebundenen APTMS-Moleküle vernetzt werden: 30 Minuten bei 90°C mit 5% APTMS oder PTMS oder TeMS in Wasser; pH 5,5 bis 5,8.

Die Konzentration der PDC kann, wie oben für das APTMS beschrieben, zwischen 0,04% und 1% variiert werden, um so die Dichte der Linker für die Aufnahme der Leit-DNA je nach Bedarf einzustellen.

Anstelle von PDC können auch andere Diisocyanate verwendet werden. PDC hat den Vorteil, daß eine Vernetzung von benachbarten Aminogruppen sterisch gehindert wird. Um einen größeren Abstand zwischen Trägeroberfläche und DNA zu erhalten, kann es von Vorteil sein, längere Verbindungsmoleküle zwischen derivatisierter Oberfläche und DNA oder mehrere Einheiten kurzer Verbindungsmoleküle hintereinander zu setzen.

Die Generierung der PCR-Fragmente erfolgt vorzugsweise, indem die Information einer mRNA mit Hilfe der reversen Transkriptasen in DNA umgeschrieben wird. Diese DNA wird mit der Polymerasen-Kettenreaktion (PCR) amplifiziert. Für beide enzymatische Vorgänge werden unter anderem Oligonukleotidprimer benötigt, die mit einer Matrize hybridisieren und als Synthesestart für die jeweilige Polymerase dienen.

Es wird eine Liste von Genen erstellt, deren parallele Identifizierung und Quantifizierung von Interesse ist. Diese Liste wird als Input für ein hierfür erstelltes Programm genutzt. Mit Hilfe des Programms werden die Sequenzen der zu analysierenden Gene z.B. einer öffentlich zugänglichen Gen-Datenbank entnommen und Oligonukleotidpaare entworfen, die eine spezifische Amplifizierung von jeweils 200 bis 400 bp Fragmenten eines jeden Gens ergeben. Die Oligonukleotide werden synthetisiert und die RT-PCR nach einem dem Fachmann an sich bekannten Protokoll durchgeführt. Die PCR-Fragmente werden zur Abtrennung nicht eingebauter Nukleotide und Oligonukleotide mit Ethanol gefällt und mit 100 mM Natriumcarbonat/Natriumhydrogencarbonat-Lösung, pH 9, eine Konzentration von 0 bis 50 ng/µl eingestellt.

## Patentansprüche

1. Träger mit an mindestens einer Hauptoberfläche des im wesentlichen planaren Trägers kovalent gebundener Oligo- oder Polynukleinsäure, dadurch gekennzeichnet, daß
die Hauptoberfläche des Trägers eine reaktive Gruppe aufweist, die mit einem bifunktionellen Spacer unter Ausbildung einer kovalenten Bindung zwischen einer funktionellen Gruppe des Spacers und der reaktiven Gruppe der Hauptoberfläche des Trägers reagiert hat,
die zweite funktionelle Gruppe des bifunktionellen Spacers mit einer der funktionellen Gruppen eines bifunktionellen Linkers reagiert und
die zweite funktionelle Gruppe des bifunktionellen Linkers mit dem Oligo- oder Polynukleotid, das kovalent gebunden werden soll, unter Ausbildung einer kovalenten Bindung am 5'- oder 3'-Terminus des Oligo- oder Polynukleotids reagiert hat.

2. Träger nach Anspruch 1, dadurch gekennzeichnet, daß das Oligo- oder Polynukleotid RNA, DNA oder PNA ist.

3. Träger nach einem der Ansprüche 1 oder 2, dadurch gekennzeichnet, daß der Träger aus Glas oder einem anderen hauptsächlich aus Siliziumoxid bestehenden Materials aufgebaut ist.

4. Träger nach Anspruch 1 bis 3, wobei der bifunktionelle Spacer die nachstehende Struktur hat
(XO)₃-Si-Y-Nu
wobei
X = C₁-C₃ Alkyl,
Y = C₂-C₄ Alkylen,
Nu = eine nukleophile Gruppe wie -NH₂, -NHR, mit
R = -CH₂-CH₂-NH₂, -CH₂-CH₂-NH -CH₂-CH₂-NH_{2,} -CO-NH₂, oder SH, ist.

5. Träger nach mindestens einem der Ansprüche 1 bis 4, wobei der Spacer Me₃OSi-CH₂-CH₂-CH₂-NH₂ ist.

6. Träger nach mindestens einem der Ansprüche 1 bis 5, dadurch gekennzeichnet, daß der Linker die folgende Struktur
E₁-A-E₂ aufweist,
wobei
E₁ und E₂ gleiche oder verschiedene elektrophile Gruppen wie
S = C = N- sind und
A = C₁-C₁₀ Alkyl, Heteroalkyl, Aryl, Heteroaryl, ist.

7. Träger nach mindestens einem der Ansprüche 1 bis 6, wobei der Linker die folgende Struktur hat
S = C = N-Phenylen-N = C = S.

8. Träger nach mindestens einem der Ansprüche 1 bis 7, wobei das Oligo- oder Polynukleotid unter Ausbildung einer kovalenten Bindung mittels einer am 3'- oder 5'-Terminus über ein Alkan mit einer Länge von 6 bis 18 Methylengruppen oder über einen Polyether von 2 bis 20 sich wiederholenden Struktureinheiten synthetisch oder über die PCR Reaktion angefügte primäre Aminogruppe mit einer funktionellen Gruppe des bifunktionellen Linkers reagiert hat.

9. Verwendung eines Trägers nach mindestens einem der Ansprüche 1 bis 8, in einem Verfahren zur Identifizierung und Quantifizierung von Polynukleotiden durch eine Markierung der zu analysierenden Polynukleotide und anschließende Hybridisierungsreaktion auf dem Träger.

10. Verfahren zur Herstellung eines Trägers nach mindestens einem der Ansprüche 1 bis 8, wobei
- der Spacer in einem polaren aprotischen Lösungsmittel auf die Hauptoberfläche des Trägers aufgebracht wird, woraufhin gegebenenfalls überschüssiger nicht abreagierter Spacer entfernt wird,
- der Linker in einem wasserfreien polaren aprotischen Lösungsmittel gelöst und zur Reaktion mit dem auf der Hauptoberfläche gebundenen Spacers gebracht wird,
- das am 5' - oder 3' -Terminus über eine Alkylengruppe mit einer Aminogruppe modifizierte Oligo- oder Polynukleotid in einem Puffer aufgenommen und auf dem Träger inkubiert wird zur Bindung des Oligo- oder Polynukleotids an eine freie Gruppe des bifunktionellen Linkers, gegebenenfalls gefolgt von einer Entfernung von überschüssigen freien Gruppen des bifunktionellen Linkers und
- das auf dem Träger gebundene Oligo- oder Polynukleotid denaturiert wird.
